# EUROPÄISCHE PATENTANMELDUNG

(11) **EP 3 549 445 A1**
(43) Veröffentlichungstag der Anmeldung: **09.10.2019**
(21) Anmeldenummer: 18166047.3
(22) Anmeldetag: 06.04.2018
(51) Int. Cl.: A01N 59/12, A01N 25/18, A01N 25/20, A01N 25/34, A01P 1/00, A01P 3/00, A01P 7/04, A61L 2/22

(54) **IODHALTIGER FORMKÖRPER ZUR LUFTREINIGUNG UND DESINFEKTION VON EINRICHTUNGEN IN DER LANDWIRTSCHAFT**

(71) Anmelder: Minea Brandenburg GmbH, 01099 Dresden (DE)
(72) Erfinder: Die Erfindernennung liegt noch nicht vor
(74) Vertreter: Gille Hrabal

(57) **Zusammenfassung**

Die vorliegende Erfindung betrifft eine Zusammensetzung zur Herstellung eines iodhaltigen Formkörpers und die Verwendung dieses iodhaltigen Formkörpers, unter Bildung eines Aerosols, zur Luftreinigung und Desinfektion von Einrichtungen in der Landwirtschaft.

## Beschreibung

Die vorliegende Erfindung betrifft einen iodhaltigen Formkörper. Des Weiteren betrifft die vorliegende Erfindung die Herstellung des iodhaltigen Formkörpers sowie dessen Verwendung zur Luftreinigung und Desinfektion, insbesondere innerhalb Einrichtungen in der Tierzucht und Landwirtschaft.

Die Verwendung von Aerosolen in der Viehzucht ist sowohl bei der Durchführung von therapeutischen und prophylaktischen Maßnahmen als auch als Desinfektionsmaßnahme bekannt. Eine der Möglichkeiten, Aerosole zu erzeugen, ist die Verwendung von aerosolbildenden Verbindungen, bei deren flammenlosen Verglimmen ein Aerosol des freizusetzenden Wirkstoffes gebildet wird. Halogenhaltige Wirkstoffe, wie iodhaltige Präparate, sind aufgrund deren desinfizierender Wirkung für solche Massenaerosolanwendungen besonders geeignet.

Die Anwendung solcher Aerosole wird zum Beispiel in den Druckschriften RU 2075974 C und RU 2097030 C beschrieben. Diese Druckschriften offenbaren iodhaltige Zusammensetzungen, die als Aerosole verwendet werden und spezifisch gegen Erkrankungen der Atemwege bei Hühnern eingesetzt werden. Ähnlich betrifft die RU 2399385 C die vorsorgliche Behandlung von Tieren und Vögeln, die mittels eines Aerosol-Generators iodhaltigen Verbindungen zum Einatmen ausgesetzt werden. Die RU 2548177 C beschreibt eine sehr komplexe Zusammensetzung, welche unter vielzähligen Bestandteilen auch eine geringe Menge an lod enthält und in Form eines Aerosols zum Schutz von Pflanzen gegen Pilze und Bakterien angewendet wird.

Auch die RU 2163146 C bezieht sich auf die vorsorgliche oder therapeutische Anwendung iodhaltiger Aerosole in der Viehzucht, sowie auch auf deren Anwendung als Desinfektionsmittel für Tierzuchtanlagen. Die Zusammensetzung, die zur Aerosolbildung verwendet wird, kann in Form einer Tablette ausgebildet sein. Nachteilig an dieser Tablettenform ist, dass größere Mengen an lod in der Zusammensetzung verwendet werden müssen, um eine ausreichende Effektivität zu erreichen. Zudem ist die Herstellung aufwendig und die resultierende iodhaltige Tablette ist nicht ausreichend lagerstabil. Dieses liegt insbesondere daran, dass der lodbestandteil mit den übrigen Bestandteilen der Tablette innig vermischt ist.

Nachteilig ist darüber hinaus das Erfordernis des Einsatzes von größeren Mengen an lod, um das gewünschte Ergebnis zu erreichen. Dieses führt zum einen zu einem hohen Kostenaufwand und zum anderen zu aufwendigen Lagerungsbedingungen. Bei der Anwendung der beschriebenen Zusammensetzungen sind ferner schädliche Auswirkungen auf die Umwelt oder die Gesundheit der Tiere nicht auszuschließen.

Darüber hinaus sind die Verfahren des Standes der Technik dahingehend nachteilig, dass die erzeugten Aerosole durch Kondensation von Dämpfen, Verdampfen flüssiger Bestandteile, Koagulation kleiner Teilchen und/oder Abscheidung von Teilchen an umgebenden Gegenständen keine homogene Verteilung im Raum gewährleisten.

Ausgehend von diesem Stand der Technik besteht eine zu lösende Aufgabe der vorliegenden Erfindung darin, eine iodhaltige Zusammensetzung in einer Verabreichungsform bereitzustellen, die für die Erzeugung eines vorzugsweise stabilen, homogenen und fein dispergierten Aerosols geeignet ist. Dabei soll insbesondere erreicht werden, dass die lodpartikel länger in einem Schwebezustand verbleiben und die Desinfektion der betreffenden Räumlichkeiten auch an schwer zugänglichen Stellen sichergestellt wird.

Eine weitere zu lösende Aufgabe der vorliegenden Erfindung besteht darin, eine iodhaltige Zusammensetzung in einer Verabreichungsform bereitzustellen, welche vorzugsweise keine aufwendige und komplizierte Herstellung erfordert, keiner aufwendigen Handhabung bedarf, lagerstabil ist und bei Anwendung keine negativen Auswirkungen auf Tiere, Einrichtungen und Umwelt aufweist.

Zur Lösung dieser Aufgaben stellt die vorliegende Erfindung einen Formkörper mit den Merkmalen des Hauptanspruchs bereit. Vorteilhafte Ausgestaltungen ergeben sich aus den Unteransprüchen.

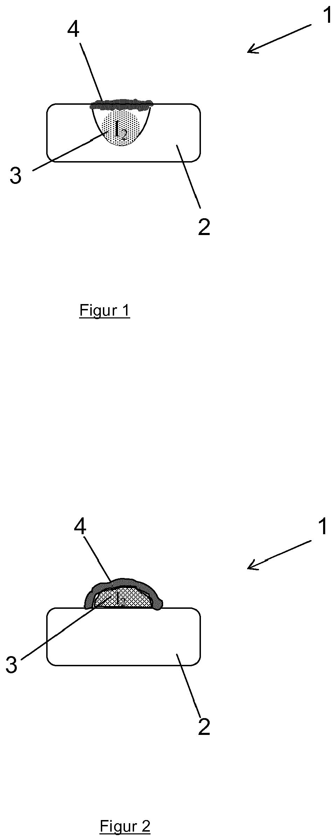

Zur Erläuterung der vorliegenden Erfindung und zur Verdeutlichung der bevorzugten Ausgestaltungen wird auch auf die beigefügten Zeichnungen (Figur 1 und 2) verwiesen.

Die vorliegende Erfindung betrifft zunächst einen Formkörper (1), der mindestens zwei Kompartments (2, 3) aufweist, wobei das erste Kompartment (2)
a) mindestens einen technischen Kohlenstoff;
b) Kalisalpeter; und
c) Magnesiumcarbonat umfasst; und
das zweite Kompartment (3) elementares lod und/oder Kaliumiodid (Kl) enthält. Darüber hinaus ist vorgesehen, dass das erste Kompartment (2) und das zweite Kompartment (3) miteinander in Kontakt stehen und dass das zweite Kompartment wenigstens teilweise räumlich mit einem Wachs (4) versiegelt ist.

Im Rahmen der vorliegenden Erfindung weist der Formkörper eine feste Form auf und umfasst die vorstehend bezeichneten Kompartments (2, 3). Unter Kompartments (2, 3) werden im Rahmen der vorliegenden Erfindung räumlich voneinander getrennte Bereiche des Festkörpers (1) verstanden, die jedoch so miteinander in Kontakt stehen, dass ein Verbrennen des Kompartments (2) zu einer Freisetzung des elementaren Iods und/oder Kaliumiodis (Kl) führt.

In einer weiteren Ausführungsform der vorliegenden Erfindung umfasst der erfindungsgemäße Formkörper (1) mindestens zwei Kompartments (2, 3), wobei das erste Kompartment (2) aus
a) mindestens einem technischen Kohlenstoff;
b) Kalisalpeter; und
c) Magnesiumcarbonat besteht.

In einer weiteren Ausführungsform der vorliegenden Erfindung umfasst der erfindungsgemäße Formkörper (1) mindestens zwei Kompartments (2, 3), wobei das zweite Kompartment (3) aus elementarem lod und/oder Kaliumiodid (Kl) besteht.

In einer weiteren Ausführungsform umfasst der erfindungsgemäße Formkörper (1) mindestens zwei Kompartments (2, 3), wobei das erste Kompartment (2) aus
a) mindestens einem technischen Kohlenstoff;
b) Kalisalpeter; und
c) Magnesiumcarbonat besteht; und
das zweite Kompartment (3) aus elementarem lod und/oder Kaliumiodid (Kl) besteht.

Der erfindungsgemäße Formkörper (1) weist zwei Kompartments (2, 3) auf. Diese Kompartments (2, 3) weisen unterschiedliche Funktionen auf. Das erste Kompartment (2), dass
a) mindestens einem technischen Kohlenstoff;
b) Kalisalpeter; und
c) Magnesiumcarbonat umfasst oder aus diesen Bestandteilen besteht
dient zunächst zum gleichmäßigen Verbrennen und damit zur Energiefreisetzung (Wärme). Diese Energie (Wärme) dient dann wiederum zum Verdampfen des iodhaltigen Bestandteils im zweiten Kompartment (3) (elementares lod bzw. Kaliumiod (Kl). Dieses funktionale Verhältnis zwischen den beiden Kompartments (2, 3) ist für die Funktionsweise des erfindungsgemäßen Formkörpers entscheidend. Die Anordnung der beiden Kompartments (2, 3) zueinander sowie die Auswahl der Bestandteile des ersten Kompartments (2) müssen so ausgewählt werden, dass bei Anwendung des erfindungsgemäßen Formkörpers ein gleichmäßiges Verdampfen (ohne Zersetzung) des iodhaltigen Bestandteils der zweiten Kompartments gewährleistet ist.

Der Ausdruck "in Kontakt stehen" bedeutet im Rahmen der vorliegenden Erfindung, dass die beiden Kompartments (2, 3) entweder unmittelbar in Kontakt stehen, d.h. sich berühren, oder mittels einer Zwischenschicht zumindest dergestalt miteinander in Kontakt stehen, dass durch die weiter unten beschriebene Art der Verwendung eine effiziente Freisetzung des lod oder Kaliumiodids gewährleistet ist.

Erfindungsgemäß eignet sich als Zwischenschicht insbesondere jede Schicht, die zur Optimierung der Verbrennung und der Aerosolbildung beitragen oder zur verbesserten Lagerstabilität dienen kann. Beispielsweise kann die Zwischenschicht, falls vorhanden, über welche die beiden Kompartments (2, 3) in Kontakt stehen, eine Wachsschicht sein.

Wie oben erwähnt gehört zu den Vorteilen der vorliegenden Erfindung auch die einfache und unkomplizierte Herstellung des erfindungsgemäßen iodhaltigen Formkörpers. Die beiden Kompartments (2, 3) können nämlich separat hergestellt und in einem weiteren Schritt zusammengeführt werden. Somit bietet sich, abgesehen von der einfachen Herstellung und Handhabung, auch eine erweiterte Kombinationsmöglichkeit der Kompartments an, wie eine Kombination verschiedener Konzentrationen der lodkomponente im zweiten Kompartment (3) mit verschiedenen Formen bzw. Größen des ersten Kompartments (2).

In einer bevorzugten Ausführungsform wird die Herstellung des Formkörpers (1) durch die folgenden Verfahrensschritte gekennzeichnet
i) Bereitstellung des ersten Kompartments (2) durch Mischen der Bestandteile (a) bis (c) und anschließendem Zusammenpressen der Mischung unter Ausbildung einer Form des Kompartments (2);
ii) Bereitstellung des zweiten Kompartments (3), umfassend mindestens elementares lod und/oder Kaliumiodid (Kl),
iii) zumindest teilweise räumliches Versiegeln des zweiten Kompartments (3) mit einem Wachs; und
iv) Inkontaktbringen des ersten Kompartments (2) mit dem zweiten Kompartment (3).

Die Reihenfolge der Verfahrensschritte kann beliebig sein; bevorzugt wird das erfindungsgemäße Verfahren in der Reihenfolge i), ii), iii) und iv) durchgeführt.

Insbesondere ist es auch möglich, dass die beiden Kompartments (2, 3) industriell separat hergestellt werden und erst kurz vor der Verwendung durch den finalen Anwender in Kontakt gebracht werden.

Gemäß der vorliegenden Erfindung kann der Formkörper jede mögliche dreidimensionale Form aufweisen. Der Formkörper kann beispielsweise eine Kugel-, Quader-, Zylinder-, Prismaform oder eine ähnliche Form aufweisen. Erfindungsgemäß kann der Formkörper abgerundet oder eckig sein. In einer bevorzugten Ausführungsform hat der Formkörper die Form einer Tablette.

In einer Ausführungsform der vorliegenden Erfindung ist das zweite Kompartment (3) auf einer Seite des ersten Kompartmens (2) vorgesehen und wird erfindungsgemäß mit Wachs abgedeckt (versiegelt). Dabei kann sich der Wachs vorzugsweise über das zweite Kompartment (3) und teilweise über das erste Kompartment (2) erstrecken.

In einer weiter bevorzugten Ausführungsform der vorliegenden Erfindung weist das erste Kompartment (2) an einer beliebigen Seite oder Stelle eine Aussparung auf. Erfindungsgemäß wird das zweite Kompartment (3) in die Aussparung des Formkörpers gefüllt und mit Wachs versiegelt. Dabei kann sich der Wachs ebenfalls vorzugsweise über das zweite Kompartment (3) und teilweise über das erste Kompartment (2) erstrecken.

Im Rahmen der vorliegenden Erfindung wird als "Iodkomponente", die das zweite Kompartment (3) ausbildet, elementares lod, Kl oder Mischungen davon verstanden.

In einer weiter bevorzugten Ausführungsform kann die lodkomponente in dem zweiten Kompartment in eine PE-Kapsel eingefüllt sein und dann in die Aussparung des Formkörpers gesteckt und mit Wachs versiegelt werden. Dadurch wird eine noch bessere Lagerstabilität des erfindungsgemäßen Formkörpers und zudem auch eine leichtere Handhabung beim Bereitstellen der beiden Kompartments (2, 3) erreicht.

Der Begriff "Versiegeln" bedeutet im Rahmen der vorliegenden Erfindung, dass eine Abdeckung des zweiten Kompartments (3), enthaltend die Iodkomponente, mittels eines Wachses erfolgt, um eine unerwünschte bzw. vorzeitige Verdampfung des Iods zu verhindern. Hierdurch wird die Lagerstabilität des erfindungsgemäßen Formkörpers wesentlich verbessert.

Der Wachs (4), welcher zur Versiegelung des zweiten Kompartments (3) benutzt wird, kann jede Art von Wachs sein. In einer Ausführungsform kann dieser Versiegelungswachs (4) ein natürlicher, pflanzlicher, tierischer, mineralischer oder synthetischer Wachs sein. In einer besonders bevorzugten Ausführungsform ist der Versiegelungswachs Stearinwachs, Paraffinwachs oder Bienenwachs.

In einer erfindungsgemäßen Ausführungsform umfasst das erste Kompartment (2) ein Zündmittel. Erfindungsgemäß kann dieses Zündmittel eine Zündschnur, ein elektrischer Zünder, ein Wendelfaden oder ähnliches sein. Durch Anzünden des ersten Kompartments (2) erfolgt eine thermische Einwirkung auf die lodkomponente des zweiten Kompartments (3), wobei entweder durch den Dampfdruck des verdampfenden Iods oder durch direktes Schmelzen des Wachses die Versiegelung zumindest teilweise aufgelöst wird und das somit verdampfende lod als Aerosol freigesetzt wird. Zum Anzünden des erfindungsgemäßen Formkörpers (1), insbesondere des ersten Kompartmenets (2), kann somit eine offene Flamme (beispielsweise eine Art von einem "Crème Brulée-Brenner"), ein Brenner mit Passform für den erfindungsgemäßen Formkörper (1) oder ein Brenner mit Anschluss für einen Wendelfaden oder eine elektrisch Zündung verwendet werden.

Die Zusammensetzung des ersten Kompartments (2) des erfindungsgemäßen Formkörpers (1) bildet eine Art pyrotechnischen Zünder aus, wobei der technischer Kohlenstoff (a) der Brennstoff ist, Kalisalpeter (b) als Sauerstoffspender für die Verbrennung dient und Magnesiumcarbonat (c) unter anderem zur Steuerung der Reaktion und zur Gewährleistung eines gleichmäßigen Abbrennens dient.

Bei dem technischen Kohlenstoff (a) handelt es sich vorzugsweise um einen gereinigten Kohlestaub. Daher trägt der technische Kohlenstoff nicht so viele Verunreinigungen und Begleitelemente mit sich wie normale Kohle, welche die Stabilität des Aerosols negativ beeinträchtigen können.

Die Stabilität des Glimm-Prozesses und der Aerosolbildung, d.h. das Verhalten des erzeugten Aerosols, hängt in der Regel von den Teilchen und dem Trägergas ab. Durch die Verbrennung des erfindungsgemäßen Formkörpers (1) bildet sich ein stabiles Aerosol, welches die aufgrund der inzwischen erhöhten Temperatur verdampfte und durch das geschmolzene Wachs entwichene lodkomponente mit sich reißt. Das Aerosol funktioniert dabei wie ein Trägergas und transportiert die Iodkomponente, welche auf diese Weise lange in der Dampfphase bzw. im Schweben verbleibt.

In einer bevorzugten Ausführungsform der vorliegenden Erfindung beträgt die Menge an technischem Kohlenstoff (a), bezogen auf die gesamte Zusammensetzung des ersten Kompartments (2) des Formkörpers (1), 10 bis 30 Gew.%. Weiter bevorzugt beträgt die Menge an technischem Kohlenstoff (a) 15 bis 25 Gew.%, noch weiter bevorzugt 18 bis 22 Gew.%, jeweils bezogen auf die gesamte Zusammensetzung des ersten Kompartments (2) des Formkörpers (1).

In einer bevorzugten Ausführungsform der vorliegenden Erfindung beträgt die Menge an Kalisalpeter (b), bezogen auf die gesamte Zusammensetzung des ersten Kompartments (2) des Formkörpers (1), 60 bis 85 Gew.%. Weiter bevorzugt beträgt die Menge an Kalisalpeter (b) 65 bis 80 Gew.%, noch weiter bevorzugt 68 bis 75 Gew.%, jeweils bezogen auf die gesamte Zusammensetzung des ersten Kompartments (2) des Formkörpers (1).

In einer bevorzugten Ausführungsform der vorliegenden Erfindung beträgt die Menge an Magnesiumcarbonat (c), bezogen auf die gesamte Zusammensetzung des ersten Kompartments (2) des Formkörpers(1), 3 bis 20 Gew.%. Weiter bevorzugt beträgt die Menge an Magnesiumcarbonat (c) 5 bis 15 Gew.%, noch weiter bevorzugt 7 bis 10 Gew.%, jeweils bezogen auf die gesamte Zusammensetzung des ersten Kompartments (2) des Formkörpers (1).

Da der erfindungsgemäße Formkörper je nach Anwendungsbereich in verschiedenen Größen hergestellt werden kann, ist auch die Menge an lod im zweiten Kompartment (3) des Formkörpers (1) abhängig von der Größe des Formkörpers (1). Das bedeutet, je größer oder verwinkelter die Räumlichkeiten sind, wo die Anwendung des erfindungsgemäßen Formkörpers (1) stattfinden soll bzw. je größer bzw. schwerer der Formkörper (1) ist, desto größer ist auch die Menge an lodkomponenten im zweiten Kompartment (3). Hier sollte allerdings erwähnt werden, dass die Energie, die aus der Verbrennung des ersten Kompartments (2) des Formkörpers zur Verfügung steht, nicht unbegrenzt ist. Das bedeutet, dass es eine maximale Menge an lodkomponente in dem zweiten Kompartment (3) gibt, welche durch die exotherme Reaktion in die Gasphase gebracht werden kann.

In einer bevorzugten Ausführungsform der vorliegenden Erfindung enthält das zweite Kompartment (3) elementares lod und die Menge an elementarem lod im zweiten Kompartment (3) des Formkörpers (1), bezogen auf das gesamte Gewicht des Formkörpers (1), beträgt vorzugsweise 0,1 bis 14 Gew.%. Weiter bevorzugt beträgt die Menge an elementarem lod im zweiten Kompartment (3) des Formkörpers (1) 2 bis 12 Gew.%, noch weiter bevorzugt 3 bis 10 Gew.%, jeweils bezogen auf das gesamte Gewicht des Formkörpers (1).

In einer weiteren Ausführungsform der vorliegenden Erfindung enthält das zweite Kompartment (3) Kaliumiodid (Kl) und die Menge an Kl im zweiten Kompartment (3) des Formkörpers (1), bezogen auf das gesamte Gewicht des Formkörpers (1), beträgt 0,1 bis 2 Gew.%. Weiter bevorzugt beträgt die Menge an Kaliumiodid (Kl) im zweiten Kompartment (3) des Formkörpers (1) 0,1 bis 1 Gew.%, noch weiter bevorzugt 0,15 bis 0,50 Gew.%, jeweils bezogen auf das gesamte Gewicht des Formkörpers (1).

In einer besonders bevorzugten Ausführungsform der vorliegenden Erfindung enthält das erste Kompartment (2) des Formkörpers (1):
72 % Kalisalpeter;
20 % technischen Kohlenstoff; und
8 % Magnesiumcarbonat,
jeweils bezogen auf die gesamte Zusammensetzung des ersten Kompartments (2) des Formkörpers (1).

Erfindungsgemäß ist der Formkörper (1) überwiegend für eine Innenraumanwendung in der Landwirtschaft vorgesehen, beispielsweise in geschlossenen Stallanlagen, Silos wie Futtersilos, Gewächshäuser, Folientunnel oder ähnliches.

Der Formkörper (1) gemäß der vorliegenden Erfindung wird als Desinfektionsmittel und/oder als Fungizid (z. B. gegen Mehltau) und/oder als Insektizid (z. B. gegen Milben, Thrips, weiße Fliege, Blattläuse) und/oder als Düngemittel verwendet. Besonders bevorzugt wird der erfindungsgemäße Formkörper (1) gegen Blutmilben bei Hühnern verwendet.

Die Häufigkeit der Anwendung hängt vom Anwendungsbereich (Größe, verwinkelte Räume usw.) und von der Art des Einsatzes (Desinfektionsmittel, Insektizid usw.) ab.

### Bezugszeichenliste:

1 erfindungsgemäßer Formkörper
2 das erste Kompartment
3 das zweite Kompartment
4 Versiegelungswachs

## Patentansprüche

1. Formkörper (1), umfassend mindestens zwei Kompartments (2, 3), wobei das erste Kompartment (2)
a) mindestens einen technischen Kohlenstoff;
b) Kalisalpeter; und
c) Magnesiumcarbonat
und das zweite Kompartment (3)
d) elementares lod und/oder
e) Kl
enthält, **dadurch gekennzeichnet, dass**
das erste Kompartment (2) und das zweite Kompartment (3) miteinander in Kontakt stehen und das zweite Kompartment (3) wenigstens teilweise räumlich mit einem Wachs (4) versiegelt ist.

2. Formkörper (1) nach Anspruch (1), **dadurch gekennzeichnet, dass** das Kompartment (2) eine Aussparung aufweist, in welche das Kompartment (3) eingelassen ist.

3. Formkörper (1) nach Anspruch 1 oder 2, **dadurch gekennzeichnet, dass** das zweite Kompartement (3) elementares Iod enthält und dass die Menge an elementarem Iod im zweiten Kompartment (3) 0,1 bis 14 Gew.%, bezogen auf das gesamte Gewicht des Formkörpers (1), beträgt.

4. Formkörper (1) nach einem der Ansprüche 1 bis 3, **dadurch gekennzeichnet, dass** das zweite Kompartment (3) Kaliumiodid (Kl) enthält und dass die Menge an Kaliumiodid (Kl) im zweiten Kompartment (3), 0 bis 2 Gew.%, bezogen auf das gesamte Gewicht des Formkörpers (1), beträgt.

5. Formkörper (1) nach einem der Ansprüche 1 bis 4, **dadurch gekennzeichnet, dass** die Menge an technischem Kohlenstoff (a) im ersten Kompartment (2) 10 bis 30 Gew.%, bezogen auf die gesamte Zusammensetzung des ersten Kompartments (2) des Formkörpers (1), beträgt.

6. Formkörper (1) nach einem der Ansprüche 1 bis 5, **dadurch gekennzeichnet, dass** die Menge an Kalisalpeter (b) im ersten Kompartment (2) 60 bis 85 Gew.%, bezogen auf die gesamte Zusammensetzung des ersten Kompartments (2) des Formkörpers (1), beträgt.

7. Formkörper (1) nach einem der Ansprüche 1 bis 6, **dadurch gekennzeichnet, dass** die Menge an Magnesiumcarbonat (c) im ersten Kompartment (2) 3 bis 20 Gew.%, bezogen auf die gesamte Zusammensetzung des ersten Kompartments (2) des Formkörpers (1), beträgt.

8. Formkörper (1) nach einem der Ansprüche 1 bis 7, **dadurch gekennzeichnet, dass** das erste Kompartment (2) ein Zündmittel umfasst.

9. Formkörper (1) nach einem der Ansprüche 1 bis 8, **dadurch gekennzeichnet, dass** der Wachs, der zur Versiegelung des zweiten Kompartments (3) verwendet wird, Stearinwachs, Paraffinwachs oder Bienenwachs ist.

10. Herstellung des Formkörpers (1) nach einem der Ansprüche 1 bis 9, **gekennzeichnet durch** die folgenden Verfahrensschritte:
i) Bereitstellung des ersten Kompartments (2) durch Mischen der Bestandteile (a) bis (c) und anschließendem Zusammenpressen der Mischung unter Ausbildung einer Form des Kompartments (2);
ii) Bereitstellung des zweiten Kompartments (3), umfassend mindestens elementares Iod und/oder Kl,
iii) zumindest teilweise räumliches Versiegeln des zweiten Kompartments mit mindestens einem Wachs; und
iv) Inkontaktbringen des ersten Kompartments (2) mit dem zweiten Komparment (3).

11. Verwendung des Formkörpers (1) gemäß einem der Ansprüche 1 bis 9 als Desinfektionsmittel und/oder Fungizid und/oder Insektizid und/oder Düngemittel.
